# NEW EUROPEAN PATENT SPECIFICATION

(11) **EP 2 204 146 B2**
(45) Date of publication and mention of the opposition decision: **02.12.2020**
(45) Mention of the grant of the patent: 14.08.2013
(21) Application number: 10002574.1
(22) Date of filing: 05.05.2005
(51) Int. Cl.: A43B 1/00, A43B 7/14, A43B 13/14, A43B 13/16, A43B 13/18

(54) **Footwear orthosis**
Fussorthese
Orthèse de pied

(30) Priority: 06.05.2004 IT MC20040063
(43) Date of publication of application: 07.07.2010
(62) Divisional of application: 05009801.1
(73) Proprietor: Salvatelli S.r.l., 63014 Montegranaro (Ascoli Piceno) (IT)
(72) Inventor: Salvatelli, Franco, 62012 Civitanova Marche (MC) (IT); Tulipani, Massimo, 6850 Medrisio (CH)
(74) Representative: Cutropia, Gianluigi

(56) References cited:
- EP-A- 0 770 368
- EP-B1- 1 209 991
- AT-U- 002 052
- DE-U1- 20 113 709
- US-A- 4 378 793
- US-A- 4 557 060
- US-A- 4 572 169
- US-A- 5 250 021
- US-A- 5 352 189
- US-A- 5 368 551
- US-A- 5 425 701
- US-A- 5 761 834
- US-A- 5 768 803
- US-A- 5 827 210
- US-A1- 2004 019 307
- US-B1- 6 453 578
- US-B1- 6 536 737
- Decision T169-83
- Decision T 398-00
- Decision T 1054-92
- Piaggesi et al: "Ann Off-the-shelf Instant Contact Casting Device for the Management of Diabetic Foot Ulcers", Diabetes care, March 2007 (2007-03),
- Salvatelli Srl: "Optima Diab", , 6 February 2005 (2005-02-06), Retrieved from the Internet: URL:www.molliter.it
- Salvatelli Srl: "History of Salvatelli Srl", , 2005, Retrieved from the Internet: URL:www.molliter.it

## Description

The present invention relates to a footwear orthosis for a user having problems with his foot, e.g. following a surgical operation or owing to a foot disease.

A number of people after undergoing a surgical operation in one or both their feet have to use a footwear orthosis, i.e. a specifically structured footwear, suitable for making it possible for the user to walk with a reasonable comfort without damaging or stressing specific sensitive foot portions. More particularly, when a patient is affected by insulin-dependant diabetes some foot tissues degenerate and diabetic ulcers are formed, and when ulceration occurs the patient cannot tolerate any exerted pressure on the foot.

Searches have been conducted to devise a footwear orthosis that makes it possible for a patient with one or both ill feet to walk without aggravating or otherwise compromising the health state of his foot or feet.

The footwear orthosis used up to now usually have an upper mounted on a sole having a front or rear raised (thicker) flat portion, a lower flat portion, and an intermediate portion which is inwardly convex. Such a configuration allows the patient's footwear to avoid during walking contact with the ground, and thus pressure transmitted to the foot, at the lower and intermediate portions thereof where his most sensitive foot parts are located.

At the current state of the art, various patients for orthopaedic footwear already exist, among which the following are worthy of note:

U.S. Patent No. 5 761 834 which describes and claims a type of footwear for diabetics or for patients with injured feet, the sole of which houses an air bladder and moveable and repositionable resilient elements. In the aforementioned patent, the area in contact with the foot has a different sloping angle between the heel and the toe and is not flat like that described and claimed in the present application in which the foot of the patient rests on a flat and stiff intermediate insole member.

U.S. Patent No. 5 368 551 which claims an ankle brace walker based on the concept of the Thomas splint, which consists of an appliance well-known in the orthopaedic sector composed of a frame placed at the sides of the ankle brace walker in order to stabilize the ankle and the shinbone and decompress the front of the foot or the heel. The concept of this patent consists of the assembling of the aforementioned easily disengageable side supports to the base of the ankle brace walker, in order to stabilize the ankle and the foot of the wearer, the contact area of which is not flat like that described and claimed in the present application.

Also U.S. Patent No. 5 250 021, which bases its concept on the application of the Thomas splint, concerns an orthosis the bearing of the weight of the lower limb of which is obtained thanks to its lateral stiffness above the two sides of the ankle.

U.S. Patent No. 2004/019,307 A1, the purpose of which is to improve the stability of the ankle, based once more on the system of the Thomas splint, provides for stiff lateral parts to support the ankle and describes and claims the area in contact with the foot with a different sloping angle between the heel and the toe, which is not flat like that described in the present application.

The Patent No. EP 0 770 368 A consists of a very stiff ankle brace walker the inside of which is equipped with air bladders that serve, once inflated, to sustain and in any case to decrease the weight of the patient in order to aid decompression.

All the above-described devices base their curative effects on the immobilization of the foot or ankle and all have provision for an insole sloping to a greater or lesser degree from the heel to the front part of the foot, in contrast with the present invention whose combination of the special shape of the sole (the central portion being flat for greater support of the weight 3 and slightly sloping towards the rear part of the sole 3c, sloping or convex the rear portion 3b and the front portion 3a) together with a flat stiff intermediate insole member 7 enables a swing or rolling movement of the foot that facilitates the walking of the patient.

Such a solution from the above-cited prior art documents, although being satisfactory from some viewpoints, prevents the patient from walking naturally, as the patient while walking has to lift the orthosis and touch down at the raised portion of the footwear, i.e. with a trim substantially normal to the soil, otherwise the orthosis must be set in an inclined trim in order to touch the soil at the orthosis tip. In both cases the resulting movements of the patient's foot are quite unnatural and uncomfortable, and will slow down the patient's advancing movement.

The main object of the present invention is to provide a footwear orthosis which prevents its user from negatively affecting a specific sensitive portion of his foot or feet, and makes it possible for him or her to walk naturally at the same time.

Another object of the present invention is to provide a footwear orthosis designed to support the user's foot in a suitable walking trim and to be used for different rehabilitation stages of a patient's foot.

Another object of the present invention is to provide a footwear orthosis which can be produced at competitive costs so as to be advantageous also from an economical point of view.

These and other objects that will better appear below are achieved by a footwear orthosis including the features of claim 1.

The footwear orthosis comprises an intermediate flat portion and a front and a rear portion which are outwardly convex, said front and rear convex portion allowing, in use, said sole member at least partly to roll between an initial ground touching position and a lifting position in which its front portion is leaving the ground.

According to the invention, the insole element comprises at least two portions engageable with one another in a puzzle-like manner.

Further features and advantages of a footwear orthosis according to the present invention will better appear from the following detailed description of some presently preferred embodiments thereof, given by way of non-limiting examples of carrying out the invention, with reference to the accompanying drawings, in which:
Figure 1 shows a perspective view with parts cut away of a footwear orthosis according to the present invention;
Figure 2 illustrates a side view of the lower portion of the footwear orthosis of Fig. 1;
Figure 3 is a bottom view of the footwear orthosis of Fig. 1;
Figures 4 and 5 are a bottom and side view, respectively, of an insole reinforcing member according to the present invention;
Figure 6 is a cross-section view taken along the line VI-VI of the insole reinforcing member in Fig. 5;
Figure 7 is a cross-section view taken along the line VII-VII of the insole reinforcing member in Fig. 5;
Figure 8 is a top view of an assembled puzzle-like insole;
Figure 9 shows a perspective view of the insole of Fig. 8 with three portions thereof reciprocally engaged in a puzzle-like manner; and
Figure 10 is a perspective view in explosion of two portions of the insole of Fig. 9.

In the accompanying drawings, the same or similar parts or components have been indicated with the same reference numerals.

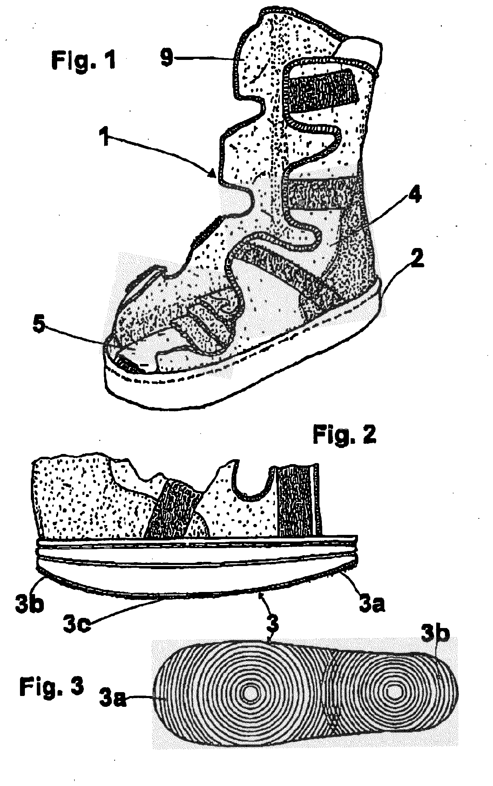

With reference first to Figures 1 to 3, it will be noted that a footwear orthosis 1 has an ambidextrous sole 2 with a tread surface 3, and an upper 4 comprising a bootleg portion.

The tread surface 3 is substantially uninterruptedly flat, i.e. a continuous flat surface substantially with no bulging or recessed portions, that can be imagined as obtained from a straight generatrix moving along a path including straight and/or two curved portions that give rise to a corresponding number of outwardly convex tread portions.

Of course, the tread surface 3 can include slightly in relief tread formations such as those generally indicated as two concentric groups of circles in Fig. 3, for proper grip with the ground.

The tread outwardly convex portions of tread surface 3, preferably have a variable radius of convexity. More particularly, the tread surface has a front portion 3a and rear portion 3b which are outwardly (downwardly, in use) convex, having a radius of convexity larger than that at the intermediate portion 3c.

The sole 2 has its upper (inner) surface 5 which is substantially flat and structured as a lattice as it is well known to a person skilled in the art. A peripheral edge 6 extends upwardly from the upper surface 5 and substantially perpendicularly to the inner surface 5, the edge 6 having a height preferably of about 45 mm.

On the upper surface a relatively rigid intermediate insole member 7 is provided which has an, in use, upper flat surface, while its lower surface in contact with the upper surface 5 of the sole has a lattice-like rib structure 7a so dimensioned as to be set in position without clearance on the lattice of the upper surface 5. The intermediate insole member 7 is preferably made of moulded antibacterical and washable ABS (acrilonitrile-butadiene-stirene) material.

A standard insole, usually bearing a trade mark, a design or the like, is provided on the insole member 7.

The footwear orthosis shown in figure 1 has a suitably shaped upper 4 with a bootleg portion and a number (four) of suitable securing means 9, e. g. each comprising a ribbon provided with an anchoring means such as a fastening means, e.g. a Velcro strip, arranged removably to secure a patient's foot to the footwear. Such a footwear orthosis makes it possible to leave the instep or upper portion of the user's foot loose and practically free from footwear portions that might exercise an undue pressure on the user's foot while walking.

During walking the user lowers the footwear orthosis at its rear portion 3b having a relatively large radius of curvature which makes it possible for the sole to effect a soft progressive rolling in the forward direction with no abrupt (torsional) stress being transmitted to the user's foot. The user's foot makes then a smooth swing movement while the footwear sole keeps in contact with the ground throughout its intermediate portion 3c and part of its front portion 3a. Once owing to rolling action the front portion 3a comes into contact with the ground the user foot is raised for a new step forward and so on.

It should be emphasized that, as an important difference with footwear orthoses according to the prior art, with a footwear orthosis according to the present invention the user touches the ground with the rear convex portion 3b of the footwear, makes a rolling movement across the intermediate portion 3c, and raises the footwear only after or upon the front convex portion 3a being rolled into contact with the ground.

The specific structure of the footwear orthosis according to the present invention thus prevents dangerous pressure picks being applied to the user's foot while the sole 2 is touching down or leaving or taking off the ground.

The touching down step is soft operation even because the rear portion 3b is then the footwear portion nearer to the ground, the user's foot being thrown forwards with its tip portion extending upwards, and thus the curved (convex) surface of the rear portion prevents the sole 2 from abruptly touching down the ground.

The same applies to lifting or take off step that takes place in an gradual and smooth way owing to the curved (convex) configuration of the front portion 3 a of the sole 2.

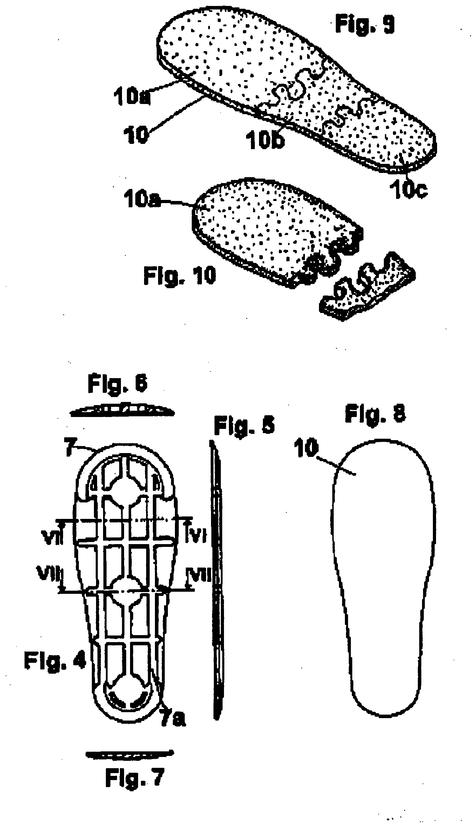

With reference now to figures 9 and 10, an insole 10 is shown which comprises three portions 10a, 10b and 10c that can be removably connected together in a puzzle-like manner.

The insole 10 is made of cushioning material and preferably an antibacteric, washable cushioning material.

The insole 10 includes a number (typically two or three) of portions 10a made of different materials thereby obtaining an insole element having mechanical characteristics changing from its rear to its front portion. Such an insole makes it possible to adapt a footwear orthosis according to the invention to specific user's needs.

Preferably, the peripheral edge 6 of the sole 2 can be dimensioned so as to make it possible to locate three or more superimposed insoles 10 in the footwear orthosis.

The footwear orthosis as above described is susceptible to numerous modifications and variations within the scope as defined by the claims.

Thus the insole can comprise portion 10a to 10c formed with an aperture or through opening to be located at sensitive portion of the user's foot.

## Claims

1. A footwear orthosis (1) including:
- sole member (2) comprising a inner surface (5) and a tread surface (3) which comprises an intermediate flat portion (3c), a outwardly convex front portion (3a) and a outwardly convex rear portion (3b), whereby said tread surface (3) can roll on the ground between a touch down position in which said rear portion (3b) comes into contact with the ground through out said intermediate portion (3c), and a take off position at said front convex portion (3a),
- a partially open upper member (4) designed to be anchored to said sole member and arranged removably to secure a user's foot thereto,
- a relatively rigid intermediate insole reinforcing member (7) located on said inner surface (5) of the sole member, and
- an insole (10) made of cushioning material located on said intermediate insole reinforcing member (7),
wherein the sole is an ambidextrous sole and said intermediate insole reinforcing member (7) has an upper flat and planar surface,
**characterized in that** said insole (10) comprises at least two portions (10a, 10b, 10c), each portion having at least an edge with protuberances and cavities so that said portions can be removably connected together in a puzzle-like manner, said portions (10a, 10b, 10c) of the insole being made of different materials, thereby obtaining an insole (10) having mechanical characteristics changing from its rear to its front portion.

2. A footwear orthosis (1) as claimed in claim 1, **characterized in that** said intermediate insole reinforcing member (7) has a lattice structure (7a) at the surface thereof designed to contact said inner surface (5) of said sole member.

3. A footwear orthosis (1) as claimed in claim 1 or 2, **characterized in that** said intermediate insole reinforcing member (7) is obtained by moulding a relatively stiff or rigid material.

4. A footwear orthosis (1) as claimed in claim 3, **characterized in that** said relatively stiff or rigid material is antibacterial acrylonitrile butadiene styrene.

## Patentansprüche

1. Schuhorthese, (1) umfassend:
- ein Sohlenelement (2) umfassend eine Innenfläche (5) und eine Lauffläche (3), die einen flachen Zwischenanteil (3c), einen vorderen, nach außen gewölbten Teil (3a) und einen hinteren, nach außen gewölbten Anteil (3b) umfasst, so dass die Lauffläche (3) zwischen einer aufliegenden Position, in der der hintere Anteil (3b) über den Zwischenanteil (3c) mit dem Boden in Kontakt kommt, und einer abgehobenen Position des vorderen gewölbten Teils (3a) auf dem Boden abrollen kann,
- ein teilweise offenes Schaftelement (4), das dazu bestimmt ist, an dem Sohlenelement verankert und abnehmbar angeordnet zu werden, um den Fuß des Trägers hiermit zu sichern,
- ein relativ starres Zwischensohlen-Verstärkungselement (7), das auf der Innenfläche (5) des Sohlenelements angeordnet ist, und
- eine Mittelsohle (10) aus weichem Material, die auf dem Zwischensohlen-Verstärkungselement (7) angeordnet ist, wobei die Sohle eine Ambidextrie-Sohle ist und das Zwischensohlen-Verstärkungselement (7) eine flache und ebene obere Fläche besitzt,
**dadurch gekennzeichnet, dass** die Mittelsohle (10) mindestens zwei Anteile (10a, 10b, 10c) umfasst, wobei jeder Anteil mindestens eine Kante mit Ausstülpungen und Hohlräumen aufweist, so dass die Anteile lösbar, in der Art eines Puzzles miteinander verbunden werden können, wobei die Anteile (10a, 10b, 10c) der Mittelsohle aus verschiedenen Materialien hergestellt sind, wodurch eine Mittelsohle (10) mit vom hinteren zum vorderen Abschnitt hin unterschiedlichen mechanischen Merkmalen erhalten wird.

2. Schuhorthese (1) nach Anspruch 1, **dadurch gekennzeichnet, dass** das Zwischensohlen-Verstärkungselement (7) eine Latexstruktur (7a) an der Oberfläche davon aufweist, die dazu bestimmt ist, mit der Innenfläche (5) des Sohlenelements in Kontakt zu kommen.

3. Schuhorthese (1) nach Anspruch 1 oder 2, **dadurch gekennzeichnet, dass** das Zwischensohlen-Verstärkungselement (7) mittels Formpressen eines relativ harten oder starren Materials hergestellt wird.

4. Schuhorthese (1) nach Anspruch 3, **dadurch gekennzeichnet, dass** das relativ harte oder starre Material ein antibakterielles Acrylnitril-Butadien-Styrolharz ist.

## Revendications

1. Orthèse (1) plantaire comprenant :
- un élément de semelle (2) incluant une surface interne (5) et une surface de semelle inférieure (3) qui comprend une portion intercalaire plate (3c), une partie antérieure convexe vers l'externe (3a) et une portion postérieure convexe vers l'externe (3b), où ladite surface de semelle inférieure (3) peut rouler sur le sol entre une position d'appui où ladite portion postérieure (3b) entre en contact avec le sol à travers ladite portion intercalaire (3c) et une position de décollage de ladite partie antérieure convexe (3a),
- un élément d'empeigne partiellement ouvert (4) destiné à être fixé au dit élément de semelle et disposé de manière amovible pour assurer le pied de l'utilisateur
- un élément de renforcement (7) de semelle intercalaire relativement rigide, positionné sur ladite surface interne (5) du ledit élément de semelle, et
- un intercalaire (10) réalisé dans un matériel souple, positionné sur ledit élément de renforcement de semelle intercalaire (7), où la semelle est une semelle du type ambidextre et que ledit élément de renforcement de semelle intercalaire (7) a une surface supérieure plate et plane.
**caractérisée en ce que** ledit intercalaire (10) comprend au moins deux parties (10a, 10b, 10c), chaque partie ayant au moins un bord avec des protubérances et des cavités, de sorte que lesdites parties peuvent s'engager l'une dans l'autre comme un puzzle, lesdites parties (10a, 10b, 10c) de l'intercalaire étant réalisées dans différentes matières afin d'obtenir un intercalaire (10) ayant des caractéristiques mécaniques qui se modifient de l'arrière vers l'avant.

2. Orthèse (1) plantaire selon la revendication 1, **caractérisée en ce que** ledit élément de renforcement de semelle intercalaire (7) a une structure réticulée (7a) sur sa surface destinée à entrer en contact avec ladite surface interne (5) du ledit élément de semelle.

3. Orthèse (1) plantaire selon la revendication 1 ou 2, **caractérisée en ce que** ledit élément de renforcement de semelle intercalaire (7) est obtenu par moulage d'un matériau relativement dur ou rigide.

4. Orthèse (1) plantaire selon la revendication 3, **caractérisée en ce que** ledit matériau relativement dur ou rigide est un latex en acrylonitrile butadiène styrène antibactérien.
